# EUROPEAN PATENT SPECIFICATION

(11) **EP 1 998 096 B1**
(45) Date of publication and mention of the grant of the patent: **28.02.2018**
(21) Application number: 08155516.1
(22) Date of filing: 30.04.2008
(51) Int. Cl.: F16L 37/098, F16L 33/22, A61M 39/12, A61M 39/10

(54) **Connector for flexible tubing**
Stecker für Schläuche
Connecteur pour tuyau flexible

(30) Priority: 15.05.2007 US 930203 P; 22.02.2008 US 66761 P
(43) Date of publication of application: 03.12.2008
(73) Proprietor: EMD Millipore Corporation, Billerica, MA 01821 (US)
(72) Inventor: Stephen, Proulx P., Boxboro, MA 01719 (US); Cianciolo, Joseph, Hudson, NH 03051 (US); Pereira, Brian, Derry, NH 03038 (US); Vigna, James, North Andover, MA 01845 (US)
(74) Representative: Henkel, Breuer & Partner

(56) References cited:
- US-A1- 2007 040 378

## Description

### FIELD OF THE INVENTION

This invention relates to a connector for connecting flexible tubing to a barbed fitting.

### BACKGROUND OF THE INVENTION

Flexible tubing is widely utilized to deliver fluid from a fluid source to a storage site or fluid treatment site. The flexible tubing is connected to the storage volume or fluid treatment site in order to provide the desired fluid delivery. It is necessary to provide a secure and leak proof connection at the end of the flexible tubing in order to avoid fluid contamination and/or leakage. Such a secure connection is particularly required in medical and pharmaceutical applications such as blood pumps, oxygen concentration cartridges, filtration cartridges, intravenous bags or the like.

At the present time, cable ties are utilized to provide a secure connection at the end of the flexible tubing. These cable ties require a tool to tighten the cable tie around the end of the flexible tubing and to cut off the excess cable tie end after the desired tightening is effected. The exposed cut cable tie end is sharp and may cause damage to the storage area, such as a flexible bag or to the fluid treatment site.

U.S. 6,796,586 B and 7,090,257 B as well as US 2005/0082826 A disclose a lock clamp for flexible tubing. The clamp requires a cumbersome tool to connect the flexible tubing to a barbed fitting.

Accordingly, it would be desirable to provide a connector for connecting a flexible tubing to a barbed fitting which prevents leakage and/or contamination of fluid located within the flexible tubing. In addition, it would be desirable to provide such a connector which remains intact even at elevated fluid pressure within the flexible tubing. Furthermore, it would be desirable to provide such a connector which can be installed by hand without the use of a tool or unusually high hand strength while avoiding the creation of sharp edges. Such a connector would provide ease of installation as well as security against fluid leakage or fluid contamination.

US 2007/0040378 A1 discloses a connector assembly including a retainer for releasably connecting a male member and a female member. The retainer has an annular housing having an outer peripheral wall defined by arms and inner retainer arms surrounding a central opening and defining with the outer arms an annular space for receiving a part of the female member whereas the male member is inserted through the central opening of the retainer. The inner arms are provided with an outward protrusion and the outer arms are provided with an inward protrusion that engage with corresponding mating recesses of the female member to provide a snap-fit connection.

### SUMMARY OF THE INVENTION

The present invention provides a connector as defined in claim 1 to connect flexible tubing to a barbed fitting. Preferred embodiments are defined in the dependent claims.

The connector comprises an annular housing section having a size to accept an open end of a flexible conduit. Flexible fingers are positioned at least on an inner peripheral surface and preferably also on an outer peripheral surface within the annular housing. The inner peripheral surface comprises one or more tabs. The tabs are sized to contact the step of a barb on a second conduit having an outer barbed surface. The second conduit is positioned within an opening formed by the inner peripheral surface (s) of the annular housing section. The fingers are sized to permit the flexible conduit to be positioned within the annular housing section and to apply pressure to the inner surface and preferably the outside surface of the flexible conduit. The barbed surface of the second conduit contacts the tab(s) when it is positioned within the opening of the connector. After connection of the flexible conduit to the second conduit is effected with the connector, removal of the flexible conduit from the connector is prevented by the fingers and removal of the second conduit from the connector is prevented by the tab(s).

In one aspect of this invention, a connector is provided having the annular housing section, the opening, the fingers and the tabs as set forth above and including a plate which exerts pressure on the outside surface of the flexible tubing, The plate can be formed in sections and can be formed integrally with the annular housing section or can comprise a separate piece which is joined to the annular housing section.

In another aspect of the present invention, if desired, the connector may be wireless enabled (such as RFID, Bluetooth® or Zigbee® devices) to help track the connector and/or the component to which it is attached.

### BRIEF DESCRIPTION OF THE DRAWINGS

Figure 1 is a top perspective view of a connector as an example showing features of this invention.
Figure 2 is a bottom perspective view of the connector of Figure 1.
Figure 3 is a cross sectional view of the connector of Figure 1.
Figure 4 is a perspective view of a barbed conduit.
Figure 5 is a cross sectional view of the connector of Figures 1-3 with a flexible conduit and a barbed second conduit positioned within the connector.
Figure 6 is a perspective view of a connector according to this invention.
Figure 7 is a cross sectional view of the connector of Figure 6 with a flexible conduit and a barbed second conduit positioned within the connector.
Figure 8 is a top view of a connector as an example showing features of this invention having hinged plate sections.
Figure 9 is a side view of the connector of Figure 8.
Figure 10 is a side view of the connector of Figure 9 having the plate sections closed.
Figure 11 is a side partial cross sectional view of the connector of Figures 8, 9 and 10 positioned on a flexible conduit and on a barb and modified with an extended surface on the plate sections.
Figure 12 is a side partial cross sectional view of an alternative example showing features of this invention having plate sections.
Figure 13 is a cross sectional view of a two piece connector as an example showing features of this invention positioned on a flexible conduit.
Figure 13a is a top-down cross sectional view of Figure 13 along lines XX to XX.
Figure 14 is a partial cross sectional view of an alternative configuration for a two piece connector as an example showing features of this invention.
Figure 15 is a partial cross sectional view of an alternative configuration of a two piece connector as an example showing features of this invention.

### DESCRIPTION OF SPECIFIC EMBODIMENTS

The connector of this invention is provided with two or more fingers which contact a flexible conduit and which are sufficiently flexible to exert pressure on the flexible conduit when a force is exerted on the flexible conduit to remove the conduit from the connector. The pressure is sufficiently high as to retain the flexible conduit within the connector or to overcome the force exerted on the flexible conduit. The fingers are sufficiently flexible so as to pivot toward the flexible conduit when a pulling force is exerted on the flexible connector which tends to move the flexible conduit out of the connector. The connector of the invention also is provided with at least one tab which fits into the step portion of a barb positioned on the outside surface of a second conduit connected to the flexible conduit through the connector. The at least one tab is sufficiently flexible so that it overrides the barb and then is snap fit into the step at the underside of the barb. It is to be understood that flexible fingers are positioned on the tabs thereby to interact with the inner surface of the flexible conduit so as to assist in retaining the flexible conduit within the connector.

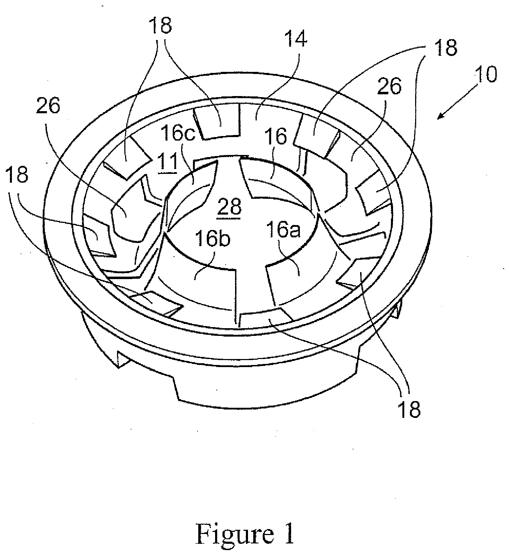

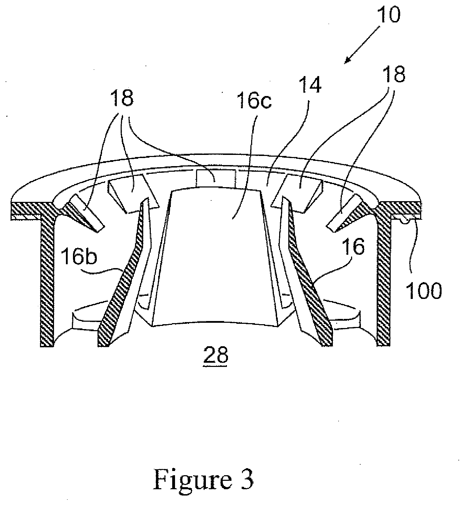

Referring to Figures 1, 2 and 3, the connector 10 of an example showing features of the invention includes an annular housing section 11 formed of outer peripheral wall 14 and the spaced apart plurality of tabs, 16, 16a, 16b and 16c. While the tabs are shown as four tabs, it is to be understood that any number of tabs can be utilized so long as they are sufficiently flexible as to override a barb positioned on the outside surface of a conduit and to snap into position into the step of the barb located at the bottom surface of the barb.

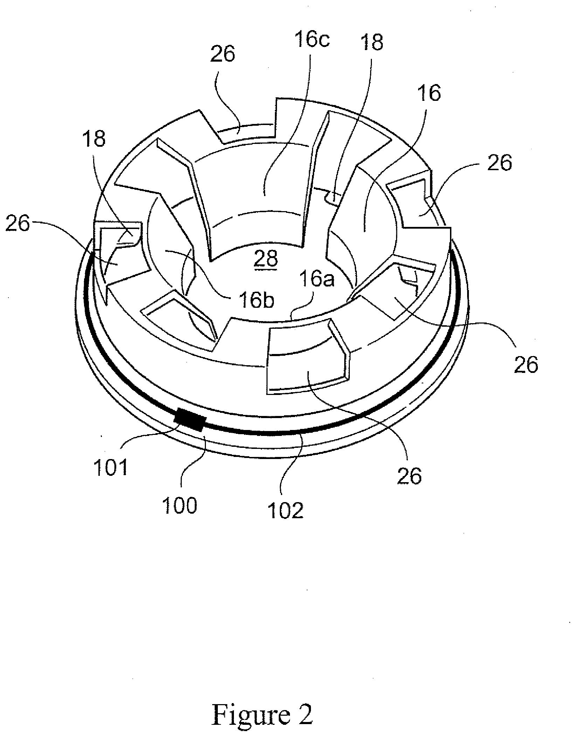

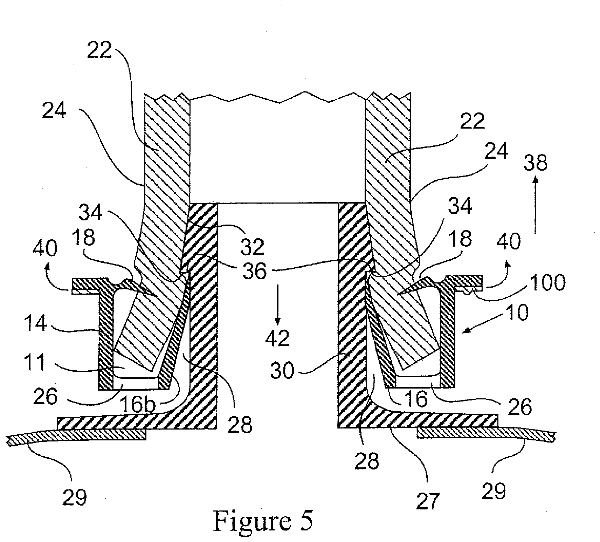

The fingers 18 are attached to the outer peripheral wall 14 and, preferably, extend inwardly from the wall 14 toward the bottom surface 20, (Figure 2) so as to provide ease of positioning the end of a flexible conduit 22 (Figure 5) into the annular section 11 of the connector 10. In addition, by extending the finger 18 downwardly, subsequent attempts to remove the flexible conduit 22 from the connector 10 are greatly diminished since the fingers 18 will flex toward the flexible conduit 22, thereby directly exerting pressure on the outside surface 24 of the flexible conduit 22 and thereby to cause the flexible conduit 22 to be retained within the connector 10. The fingers 18 can be the same length or different lengths. For example, when eight fingers 18 are employed every other finger can be the right length for a thin wall conduit and the others having an appropriate length for a thick wall conduit. Any number of fingers 18 can be used of one or more lengths so long as they are sufficient in number and length to grasp and hold the flexible conduit 22 as desired.

The connector 10 is optionally provided with spaced-apart openings 26 to increase the flexibility of the annular housing section 11 thereby to improve the ease of positioning the flexible conduit 22 into the connector 10. The hollow inner pathway 28 of the connector 10 is provided to permit the insertion of the second conduit 30 having the barb 32 having a step 34 and a bottom surface 36 (Figure 5), into which tabs 16, 16a, 16b and 16c are positioned (Figures 1 and 5), and to allow for liquid or gas to pass between the flexible conduit 22 and the second conduit 30.

Optionally, the connector 10 may be wirelessly enabled as shown in Figure 2 and other Figures described below. The wireless communications device 100 maybe a RFID tag having a communication and storage or memory component 101 and an antenna 102 as shown or other wireless devices such as Bluetooth® or Zigbee® wireless enabled communications devices.

By wirelessly enabling the connector 10 one can track the history of the connector and/or the component to which it is attached. For example, with a read only wireless device one can track the manufacture of the connector such as the lot number, date of manufacture and the like. With a read/write device containing an active memory, one can also add information to the wireless device such as when the connector was placed on the component, what the component is to which the device 100 is attached, what the component is meant to be used with, one or more trackable events that occur to the connector and the component to which it is attached such as sterilization, warehousing, use and the like.

Optionally, the wireless device may be gamma radiation stable such that the device is not damaged or destroyed due to the radiation typically used in many sterilization processes. Such devices are known as FRAM RFID and can have a storage component that employs a non-charge based storage mechanism such as a ferro-magnetic or magnetoresistive memory storage device.

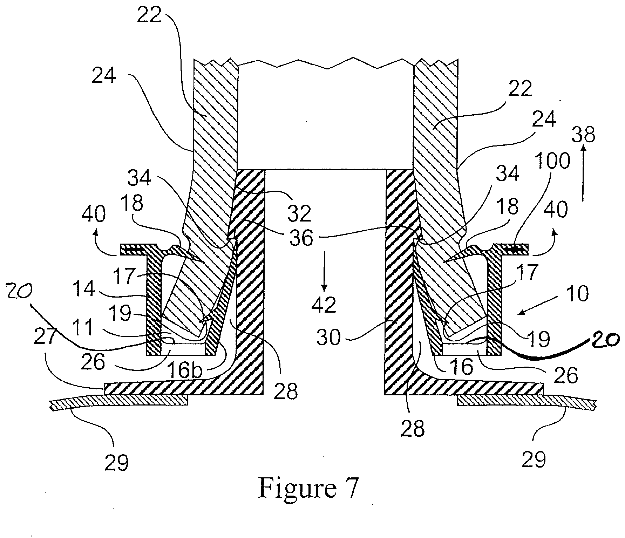

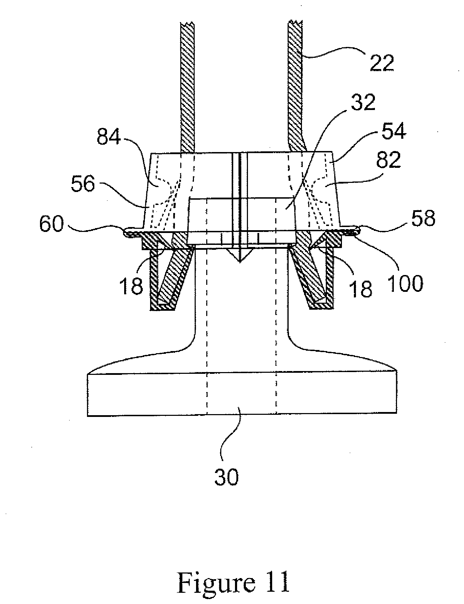

The wireless device 100 may attached to the connector by a mechanical device such as by a rivet or screw or a strap under a top surface of the connector and passing through two of the openings 26 and then to the wireless device (not shown) or it 100 can be molded into the connector 10 (as in Figures 7 and 11) or it 100 can be formed on or adhered to the surface of the connector 10 as shown in Figures 2, 3, 5 and 6.

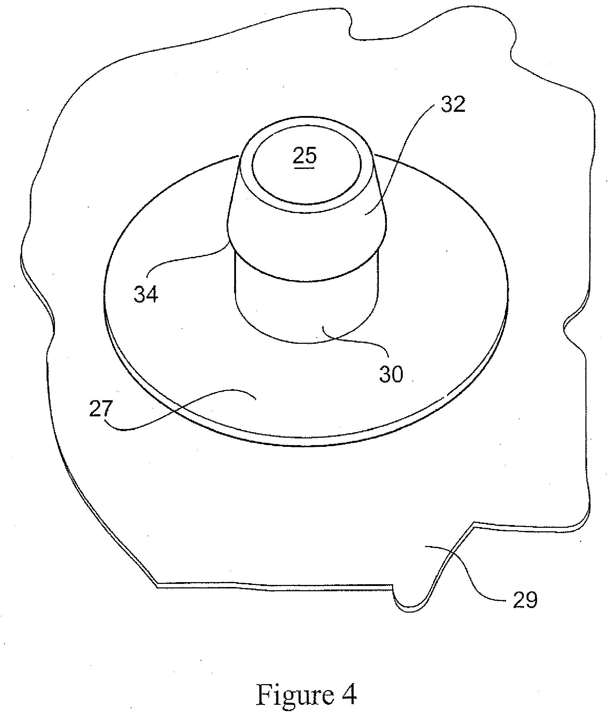

As shown in Figure 4, a barbed second conduit 30 includes a barb 32 and an opening 25 that permits fluid flow therethrough. The conduit section 30 is attached to a flange 27 which, in turn, is attached to a fluid processor 29 which can retain fluid such as a bag or can effect a unit operation such as a filtration cartridge.

As shown in Figure 5, the fingers 18 exert pressure on the outside surface 24 of flexible conduit 22. When a pulling force exemplified by arrow 38 is exerted on conduit 22, the fingers 18 pivot in the direction exemplified by arrows 40 thereby compressing the outside surface 24 of the flexible conduit 22, causing the flexible conduit 22 to be retained within the connector 10.

When a pulling force, as exemplified by arrow 42 is exerted on conduit 30, the tabs 16, 16a, 16b, and 16c exert a counter force on the bottom surface 36 of barb 32, thereby to effect retention of the conduit 30 in connector 10. Thus, the fingers 18 and tabs 16, 16a, 16b and 16c work in concert to retain the flexible conduit 22 and/or conduit 30 in the connector 10 when a pulling force is exerted on the flexible conduit 22 and top conduit 30. In addition, the positioning of conduit 22 and conduit 30 in connector 10 can be effected by hand without the need for a tool. Furthermore, the connector 10 can be sized to accept a wide size range of flexible conduits and second conduits having a barbed outer surface by providing a size range of connectors 10 having a variety of sizes of annular housing sections 11 and a variety of sizes of holes 28.

The conduit 22 has a flexibility sufficient to permit the fingers 18 to exert a pressure thereon when a force is exerted on the flexible conduit 22 in a direction to pull the flexible conduit 22 from the connector 10. Representative suitable flexible connectors can be made from silicone, preferably platinum cured silicone; polyethylene, propropylene; polyvinyl chloride; a thermoplastic elastomer; PTFE resin; EPDM, C-Flex® resin available from Consolidated Polymer Technologies of Clearwater Florida or the like. The flexible tubing may also have a protective/pressure resistive braid over them or incorporated as a jacket onto them. Such braids are well known and can be made of polyester, polypropylene or stainless steel.

The barbed conduit 30 can be made of any material such as a polymeric composition, or a metal composition such as stainless steel so long as the tabs 16, 16a, 16b and 16c can be positioned on the bottom surface 36 of the barb 34 when the barbed conduit 30 is inserted in hole 28.

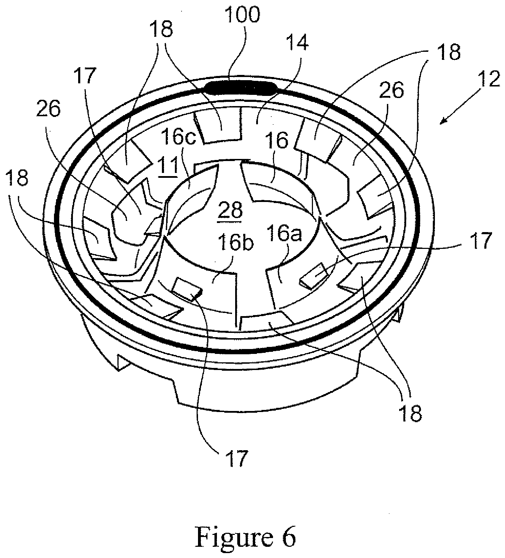

Referring to Figure 6, a connector 12 of this invention is shown. The connector 12 has the same elements of the connector 10 of Figure 1 wherein like indicia identify like elements. The connector 12 includes a second set of fingers 17 which are positioned on the tabs 16, 16a, 16b and 16c. The fingers 17 function in the same manner as fingers 18 as described above. It is to be understood that the connector can be formed with only fingers 17, without fingers 18.

As shown in Figure 7, the fingers 18 exert pressure on the outside surface 24 of flexible conduit 22 and the fingers 17 exert pressure on the inside surface 19 of flexible conduit 22. When a pulling force exemplified by arrow 38 is exerted on conduit 22, the fingers 17 and 18 pivot in the direction exemplified by arrows 40 thereby compressing the outside surface 24 and the inside surface 19 of the flexible conduit 22, causing the flexible conduit 22 to be retained within the connector 10.

When a pulling force, as exemplified by arrow 42 is exerted on conduit 30, the tabs 16, 16a, 16b, and 16c exert a counter force on the bottom surface 34 of barb 32, thereby to effect retention of the conduit 30 in connector 10. Thus, the fingers 17 and 18 and tabs 16, 16a, 16b and 16c work in concert to retain the flexible conduit 22 and/or conduit 30 in the connector 10 when a pulling force is exerted on the flexible conduit 22 and second conduit 30. The positioning of flexible conduit 22 and second conduit 30 in connector 10 can be effected by hand without the need for a tool. Furthermore, the connector 10 can be sized to accept a wide size range of flexible conduits and second conduits having a barbed outer surface by providing a size range of connectors 10 having a variety of sizes of annular housing sections 11 and a variety of sizes of holes 28.

Referring to Figures 5 and 7, in use, the barbed second conduit 30 is inserted into inner pathway 28 so that the barb 34 is positioned on the top of tabs 16a, 16b and 16c (Figures 1 and 6). The flexible conduit 22 then is inserted into annular housing section 11 to an extent such that its bottom end by-passes both sets of fingers 17 and 18. The flexible conduit 22 and second conduit 30 are thus retained within the connector 10 or 12 in the manner described above.

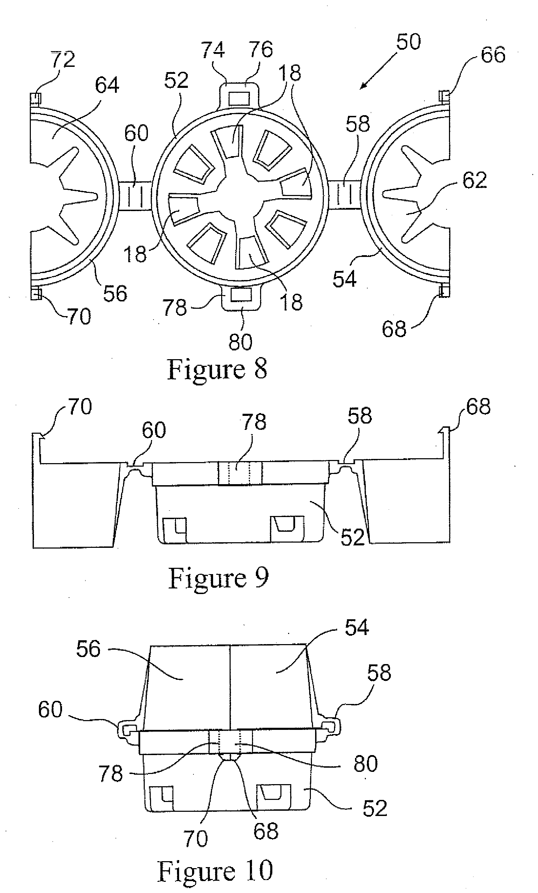

Referring to Figures 8, 9 and 10, a connector as an example showing features of this invention 50 includes a connector section 52 and two plate sections 54 and 56. The plate sections 54 and 56 are joined to connector section 52 by living hinges 58 and 60. The hinges 58 and 60 permit moving the plate sections 54 and 56 into locked contact with the connector section 52. After the flexible conduit such as flexible conduit 22 (Figure 5) is positioned within the connector section 52 as described above with reference to Figure 5, the plate sections 54 and 56 are pivoted about hinges 58 and 60. The hinges 58 and 60 function to expose the inner surface of the connector section 52 so that an end of a flexible conduit can be inserted therein. The hinges 58 and 60 also permit the plate sections 54 and 56 to be positioned in contact with an outside surface of a flexible conduit positioned within connector section 52 thereby to assist in preventing removal of the flexible conduit from the connector section 52. The plate sections 54 and 56 are locked into position against the outside surface of the flexible conduit 22 (Figure 5) so that the inside surfaces 62 and 64 press against the outside surface 24 of conduit 22 (Figure 5). It is to be understood that surfaces 62 and 64 can be smooth or rough such as serrated or having prongs extended there from to provide a gripping force on the flexible conduit. Locking is effected, for example, by means of tabs 66, 68, 70 and 72 which lock into the walls of openings 74, 76, 78 and 80. It is to be understood that locking of the plate sections 54 and 56 to connection section 52 can be effected by any conventional means. It is to be understood that more than two hinged plate sections can be utilized such as three or four plate sections. It is also to be understood that the plate sections 54 and 56 can be pivotally connected to the connector section 52 by any conventional means such as plastic ties which extend through openings shaped like openings 74, 76, 78 and 80.

As shown in Figure 11, the connector of Figures 8, 9 and 10 can be modified so that the inside surfaces of the plate sections 54 and 56 include bead shaped extended surfaces 82 and 84. The purpose of the extended surfaces 82 and 84 is to exert a compressive force on flexible conduit 22 against barb 32.

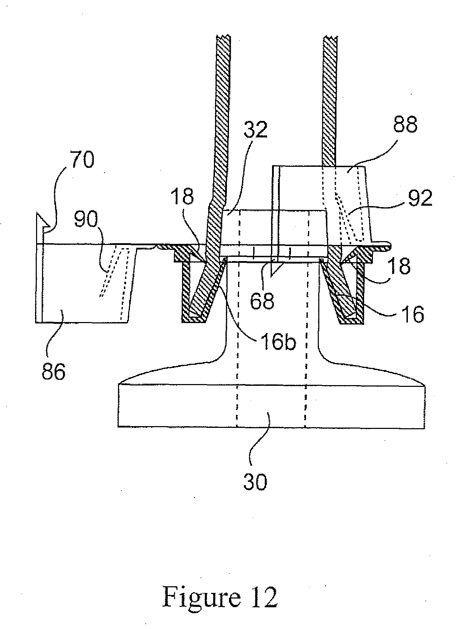

Referring to Figure 12, one alternative connector as an example showing features of this invention is shown in position on a barbed conduit. The tabs 16 and 16b function in the same manner as described above with reference to Figures 1, 2 and 3. The plate sections 86 and 88 are provided with flexible caps 90 and 92. The flexible caps 90 and 92 provide flexibility for accommodating various sized flexible conduits that are positioned with the barbed conduit 30 in the manner described above (Figure 5).

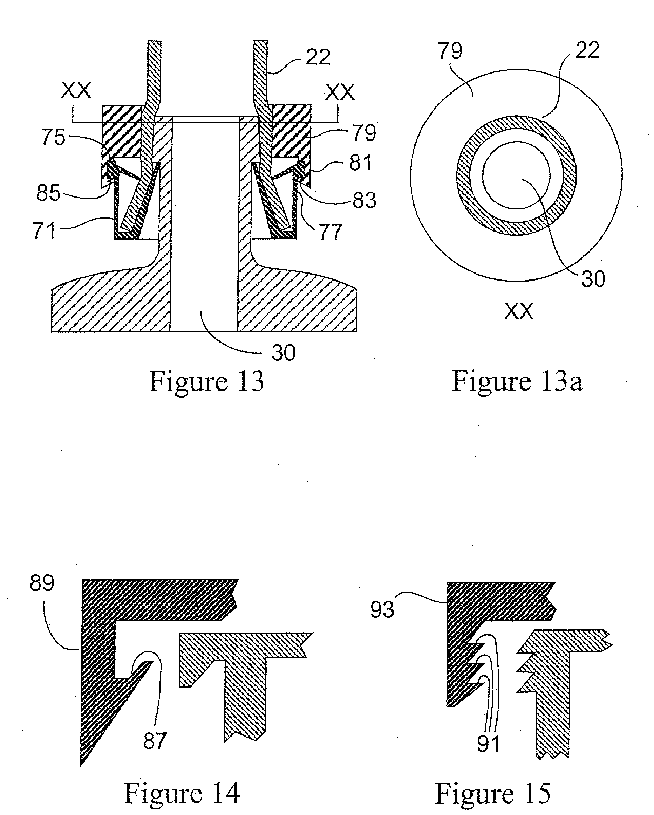

Referring to Figure 13, a two piece connector as an example showing features of this invention is shown. The connector section 71 is the same as connector 10 (Figure 1) except that it includes, on its outside surface a plurality of slots, at least two, preferably three or more, such as four slots 73 on its top surface 75 as shown. The slots 73 communicate with a circular path 77 that extends around at least a portion of the circumference of connector section 71. A second piece of this connector comprises a ring 79 which includes prongs 81 having a step 83 which fits below lip 85 of connector section 71. In use, the ring 79 is positioned on the flexible conduit 22. The end of the flexible conduit is placed in the connector section 71 in the manner described above with reference to connector 10 of Figure 5. The ring 79 then is moved into the connector section 71 by positioning the prongs 81 into the slots 73 so that the steps 83 are positioned below lip 85. The ring 79 then is rotated in circular path 77 so that the ring 79 is prevented from separating from connector section 71 by the mating lip 85 and steps 83. It is to be understood that ring 79 and connector piece 71 can be connected to each other by any conventional means such as by being snap fit together or secured to each other with conventional mating helical paths.

As shown in Figure 14, the steps on the prongs 89 can be angled. As shown in Figures 15, a plurality of angled steps 91 can be utilized on each prong 93. Any geometry which promotes retention of the ring and connector section of the two piece connector of this invention can be utilized herein.

## Claims

1. A connector for connecting a flexible first conduit (22) and a second conduit (30) having a barbed outer surface, said connector comprising:
an annular housing section (11) having an outer peripheral wall (14) defining a space for receiving the flexible first conduit (22),
one or more tab(s) (16) positioned at an inner wall portion of said annular housing section (11) and arranged outside of and adjacent to a central opening (28) through which the second conduit (30) can be inserted for securing the second conduit (30) within the connector by exerting a force on a surface (36) of a step part (34) of the barbed outer surface, and
a plurality of first fingers (17) for interacting with an inner surface (19) of said flexible first conduit (22) when the same is inserted in the space of said annular housing section (11),
wherein said first fingers (17) extend outwardly from an outer surface of one or more of said tab(s) (16) into said space for receiving the flexible first conduit (22), and **characterised in that** said first fingers further extend toward a bottom surface (20) of the space of the annular housing section (11) in the direction of insertion of the flexible first conduit (22).

2. The connector of claim 1, further comprising:
a plurality of second fingers (18) which extend from an inner surface of said outer peripheral wall (14) of said annular housing section (11) toward the bottom surface (20) of the space of the annular housing section (11) so as to interact with the outer surface (24) of the flexible first conduit (22) when the same is inserted in the space of said annular housing section (11).

3. The connector of claim 1 or 2, wherein said one or more tab(s) (16) is/are flexible.

4. The connector of any one of claims 1 to 3, further comprising means for exerting a compressive force on the outer and/or inner surface of the flexible conduit (22).

5. The connector of claim 4, wherein said means for exerting a compressive force on an outer surface of the flexible conduit (22) includes a detachable ring (79).

6. The connector of claim 5, wherein said ring (79) is attachable to said annular housing section (71).

7. The connector of claim 4, wherein said means for exerting a compressive force on an outer surface of the flexible conduit (22) includes pivotable plate sections (54,56).

8. The connector of claim 7, wherein said plate sections (54,56) are attached to said annular housing section (52) by hinges.

9. The connector of claim 8, further comprising means (66,68,70,72,74,76,78,80) for locking the plate sections (54,56) into position in contact with the outer surface of the flexible first conduit (22) and to the annular housing section (52)

10. The connector of any one of claims 1 to 9, further comprising a wireless enabled communication and memory device.

11. The connector of claim 10, wherein the wireless enabled communication and memory device is attached to it by a means selected from the group consisting of mechanical, adhesive, thermal bonding means, and moulding onto or into one of its surfaces.

12. The connector of claim 10 or 11, wherein the wireless enabled communication and memory device has read/write capability and/or is adapted to track one or more event(s) that occur(s) to the connector and/or a component to which it is connected.

13. The connector of claim 10, 11 or 12, wherein the wireless enabled communication and memory device is selected from the group consisting of RFID tags, Bluetooth® devices and Zigbee® devices.

14. A connector system for transporting fluid between a flexible first conduit (22) and a second conduit (30) having a barbed outer surface, comprising:
the flexible first conduit(22);
the second conduit (30) having the barbed outer surface; and
a connector as defined in any one of claims 1 to 13 for connecting said flexible first conduit (22) and said second conduit (30).

15. A process of securing a flexible first conduit (22) having an outer wall and an inner pathway therethrough to a second conduit (30) having a flange portion (27) and an outer barbed portion (34) which extends away from at least one side of the flange portion and which has a step part and a first opening in the flange portion and a second opening in the barbed portion (34) with a bore between the first and second openings, the process comprising the steps of:
providing a connector as defined in any one of claims 1 to 13, which connector has the central opening (28) with an inner diameter that is greater to or equal to the outer diameter of the flexible conduit (22);
inserting the flexible conduit (22) into the space defined in the annular housing section (11); and
inserting the barbed portion (34) of the second conduit (30) into the inner pathway of the flexible conduit (22) so as to cause the one or more tabs (16) to interact with the barbed portion (34) of the second conduit (30) and the one or more fingers (17,18) to interact with a surface of the flexible conduit (22) to produce a strong seal between the second conduit (30), the connector and the flexible first conduit (22).

## Patentansprüche

1. Ein Verbinder zum Verbinden einer flexiblen ersten Leitung (22) und einer zweiten Leitung (30), die eine mit Widerhaken versehene Außenfläche hat, wobei der Verbinder aufweist:
einen ringförmigen Gehäuseabschnitt (11) mit einer Außenumfangswand (14), die einen Raum zur Aufnahme der flexiblen ersten Leitung (22) definiert,
eine oder mehrere Lasche(n) (16), die an einem Innenwandabschnitt des ringförmigen Gehäuseabschnitts (11) positioniert und außerhalb von und angrenzend an eine zentrale Öffnung (28) angeordnet ist/sind, durch welche die zweite Leitung (30) zum Sichern der zweiten Leitung (30) in dem Verbinder durch Ausüben einer Kraft auf eine Oberfläche (36) eines Stufenteils (34) der mit Widerhaken versehenen Außenfläche eingesetzt werden kann, und
eine Vielzahl von ersten Fingern (17) zum Interagieren mit einer Innenfläche (19) der flexiblen ersten Leitung (22), wenn dieselbe in den Raum des ringförmigen Gehäuseabschnitts (11) eingesetzt ist,
wobei die ersten Finger (17) sich von einer Außenfläche von einer oder mehreren der Lasche(n) (16) nach außen in den Raum zur Aufnahme der flexiblen ersten Leitung (22) erstrecken, und
**dadurch gekennzeichnet, dass**
die ersten Finger sich weiter zu einer Bodenfläche (20) des Raums des ringförmigen Gehäuseabschnitts (11) in der Einsetzrichtung der flexiblen ersten Leitung (22) erstrecken.

2. Der Verbinder gemäß Anspruch 1, ferner mit:
einer Vielzahl von zweiten Fingern (18), die sich von einer Innenfläche der Außenumfangswand (14) des ringförmigen Gehäuseabschnitts (11) zu der Bodenfläche (20) des Raums des ringförmigen Gehäuseabschnitts (11) so erstrecken, dass sie mit der Außenfläche (24) der flexiblen ersten Leitung (22) interagieren, wenn dieselbe in den Raum des ringförmigen Gehäuseabschnitts (11) eingesetzt ist.

3. Der Verbinder gemäß Anspruch 1 oder 2, wobei die eine oder die mehreren Lasche(n) (16) flexibel ist/sind.

4. Der Verbinder gemäß einem der Ansprüche 1 bis 3, ferner mit einem Mittel zum Ausüben einer Kompressionskraft auf die Außen- und/oder Innenfläche der flexiblen Leitung (22).

5. Der Verbinder gemäß Anspruch 4, wobei das Mittel zum Ausüben einer Kompressionskraft auf eine Außenfläche der flexiblen Leitung (22) einen abnehmbaren Ring (79) aufweist.

6. Der Verbinder gemäß Anspruch 5, wobei der Ring (79) an dem ringförmigen Gehäuseabschnitt (71) anbringbar ist.

7. Der Verbinder gemäß Anspruch 4, wobei das Mittel zum Ausüben einer Kompressionskraft auf eine Außenfläche der flexiblen Leitung (22) schwenkbare Plattenabschnitte (54,56) aufweist.

8. Der Verbinder gemäß Anspruch 7, wobei die Plattenabschnitte (54, 56) an dem ringförmigen Gehäuseabschnitt (52) durch Gelenke bzw. Scharniere angebracht sind.

9. Der Verbinder gemäß Anspruch 8, ferner mit Mitteln (66,68,70,72,74,76,78,80) zum Verriegeln der Plattenabschnitte (54,56) in Position in Kontakt mit der Außenfläche der flexiblen ersten Leitung (22) und dem ringförmigen Gehäuseabschnitt (52).

10. Der Verbinder gemäß einem der Ansprüche 1 bis 9, ferner mit einer drahtlosen Kommunikations- und Speichervorrichtung.

11. Der Verbinder gemäß Anspruch 10, wobei die drahtlose Kommunikations- und Speichervorrichtung daran durch ein Mittel angebracht ist, das aus der Gruppe ausgewählt ist, die aus mechanischen, adhäsiven, thermischen Verbindungsmitteln und An- oder Einformen an bzw. in eine von deren Oberflächen besteht.

12. Der Verbinder gemäß Anspruch 10 oder 11, wobei die drahtlose Kommunikations- und Speichervorrichtung Schreibe/Lese-Fähigkeit hat und/oder eingerichtet ist, um ein oder mehrere Ereignis(e) zu verfolgen, das/die bezüglich des Verbinders und/oder einer Komponente, mit der er verbunden ist, auftritt/auftreten.

13. Der Verbinder gemäß Anspruch 10, 11 oder 12, wobei die drahtlose Kommunikations- und Speichervorrichtung aus der Gruppe ausgewählt ist, die aus RFID-Tags, Bluetooth®-Vorrichtungen und Zigbee®-Vorrichtungen besteht.

14. Ein Verbindersystem zum Transportieren von Fluid zwischen einer flexiblen ersten Leitung (22) und einer zweiten Leitung (30), die eine mit Widerhaken versehene Außenfläche besitzt, mit:
der flexiblen ersten Leitung (22),
der zweiten Leitung (30) mit der mit Widerhaken versehenen Außenfläche, und
einem Verbinder gemäß einem der Ansprüche 1 bis 13 zum Verbinden der flexiblen ersten Leitung (22) und der zweiten Leitung (30).

15. Ein Verfahren zum Sichern einer flexiblen ersten Leitung (22) mit einer Außenwand und einem inneren Durchgangsweg durch sie hindurch an einer zweiten Leitung (30) mit einem Flanschabschnitt (27) und einem äußeren, mit Widerhaken versehenen Abschnitt (34), der sich von mindestens einer Seite des Flanschabschnitts weg erstreckt und der ein Stufenteil und eine erste Öffnung in dem Flanschabschnitt und eine zweite Öffnung in dem mit Widerhaken versehenen Abschnitt (34) mit einer Bohrung zwischen den ersten und zweiten Öffnungen aufweist, wobei das Verfahren die folgenden Schritte aufweist:
Vorsehen eines Verbinders gemäß einem der Ansprüche 1 bis 13, welcher Verbinder die zentrale Öffnung (28) mit einem Innendurchmesser hat, der größer oder gleich dem Außendurchmesser der flexiblen Leitung (22) ist,
Einsetzen der flexiblen Leitung (22) in den in dem ringförmigen Gehäuseabschnitt (11) definierten Raum, und
Einsetzen des mit Widerhaken versehenen Abschnitts (34) der zweiten Leitung (30) in den inneren Durchgangsweg der flexiblen Leitung (22), um zu bewirken, dass die eine oder mehreren Lasche(n) (16) mit dem mit Widerhaken versehenen Abschnitt (34) der zweiten Leitung (30) interagieren, und der eine oder die mehreren Finger (17,18) mit einer Oberfläche der flexiblen Leitung (22) interagieren, um eine starke Abdichtung zwischen der zweiten Leitung (30), dem Verbinder und der flexiblen ersten Leitung (22) herzustellen.

## Revendications

1. Connecteur pour raccorder un premier conduit flexible (22) et un second conduit (30) ayant une surface externe à barbelures, ledit connecteur comprenant :
une section de logement annulaire (11) ayant une paroi périphérique externe (14) définissant un espace pour recevoir le premier conduit flexible (22),
une ou plusieurs languettes (16) positionnées au niveau d'une partie de paroi interne de ladite section de logement annulaire (11) et agencées à l'extérieur de et de manière adjacente à une ouverture centrale (28) à travers laquelle le second conduit (30) peut être inséré pour fixer le second conduit (30) à l'intérieur du connecteur en exerçant une force sur une surface (36) d'une partie de gradin (34) de la surface externe à barbelures, et
une pluralité de premiers doigts (17) pour interagir avec une surface interne (19) dudit premier conduit flexible (22) lorsque ce dernier est inséré dans l'espace de ladite section de logement annulaire (11),
dans lequel lesdits premiers doigts (17) s'étendent vers l'extérieur à partir d'une surface externe des une ou plusieurs desdites languettes (16) dans ledit espace pour recevoir le premier conduit flexible (22), et
caractérisé en ce qui :
lesdits premiers doigts s'étendent en outre vers une surface inférieure (20) de l'espace de la section de logement annulaire (11) dans la direction d'insertion du premier tuyau flexible (22).

2. Connecteur selon la revendication 1, comprenant en outre :
une pluralité de seconds doigts (18) qui s'étendent à partir d'une surface interne de ladite paroi périphérique externe (14) de ladite section de logement annulaire (11) vers la surface inférieure (20) de l'espace de la section de logement annulaire (11) afin d'interagir avec la surface externe (24) du premier conduit flexible (22) lorsque ce dernier est inséré dans l'espace de ladite section de logement annulaire (11).

3. Connecteur selon la revendication 1 ou 2, dans lequel lesdites une ou plusieurs languettes (16) est/sont flexible(s).

4. Connecteur selon l'une quelconque des revendications 1 à 3, comprenant en outre un moyen pour exercer une force de compression sur la surface externe et/ou interne du conduit flexible (22).

5. Connecteur selon la revendication 4, dans lequel ledit moyen pour exercer une force de compression sur une surface externe du conduit flexible (22) comprend une bague détachable (79).

6. Connecteur selon la revendication 5, dans lequel ladite bague (79) peut être fixée sur ladite section de logement annulaire (71).

7. Connecteur selon la revendication 4, dans lequel ledit moyen pour exercer une force de compression sur une surface externe du conduit flexible (22) comprend des sections de plaque pivotantes (54, 56).

8. Connecteur selon la revendication 7, dans lequel lesdites sections de plaque (54, 56) sont fixées sur ladite section de logement annulaire (52) par des charnières.

9. Connecteur selon la revendication 8, comprenant en outre un moyen (66, 68, 70, 72, 74, 76, 78, 80) pour bloquer les sections de plaque (54, 56) en position, en contact avec la surface externe du premier conduit flexible (22) et sur la section de logement annulaire (52).

10. Connecteur selon l'une quelconque des revendications 1 à 9, comprenant en outre un dispositif de communication et de mémoire sans fil.

11. Connecteur selon la revendication 10, dans lequel le dispositif de communication et de mémoire sans fil est fixé à ce dernier par un moyen sélectionné dans le groupe comprenant un moyen de liaison mécanique, adhésive, thermique et se moulant sur ou dans l'une de ses surfaces.

12. Connecteur selon la revendication 10 ou 11, dans lequel le dispositif de communication et de mémoire sans fil a une capacité de lecture / écriture et/ou est adapté pour tracer un ou plusieurs événements qui se produisent sur le connecteur et/ou un composant auquel il est raccordé.

13. Connecteur selon la revendication 10, 11 ou 12, dans lequel le dispositif de communication et de mémoire sans fil est sélectionné dans le groupe comprenant les étiquettes RFID, les dispositifs Bluetooth® et les dispositifs Zigbee®.

14. Système de connecteur pour transporter un fluide entre un premier conduit flexible (22) et un second conduit flexible (30) ayant une surface externe à barbelures, comprenant :
le premier conduit flexible (22) ;
le second conduit (30) ayant la surface externe à barbelures ; et
un connecteur selon l'une quelconque des revendications 1 à 13 pour raccorder ledit premier conduit flexible (22) et ledit second conduit flexible (30).

15. Procédé pour fixer un premier conduit flexible (22) ayant une paroi externe et une voie de passage interne à travers ce dernier, à un second conduit (30) ayant une partie de bride (27) et une partie externe à barbelures (34) qui s'étend à distance d'au moins un côté de la partie de bride et qui a une partie de gradin et une première ouverture dans la partie de bride et une seconde ouverture dans la partie à barbelures (34) avec un alésage entre les première et seconde ouvertures, le procédé comprenant les étapes suivantes :
prévoir un connecteur selon l'une quelconque des revendications 1 à 13, lequel connecteur a une ouverture centrale (28) avec un diamètre interne qui est supérieur ou égal à un diamètre externe du conduit flexible (22) ;
insérer le conduit flexible (22) dans l'espace défini dans la section de logement annulaire (11) ; et
insérer la partie à barbelures (34) du second conduit (30) dans la voie de passage interne du conduit flexible (22) afin d'amener les une ou plusieurs languettes (16) à interagir avec la partie à barbelures (34) du second conduit (30) et les un ou plusieurs doigts (17, 18) à interagir avec une surface du conduit flexible (22) pour produire un joint d'étanchéité robuste entre le second conduit (30), le connecteur et le premier conduit flexible (22).
